(19) **Europäisches Patentamt**
**European Patent Office**
**Office européen des brevets**

(11) **EP 4 105 940 A1**

(12) # EUROPEAN PATENT APPLICATION

(43) Date of publication:
**21.12.2022 Bulletin 2022/51**

(21) Application number: **21180215.2**

(22) Date of filing: **18.06.2021**

(51) International Patent Classification (IPC):
**G16H 40/63** (2018.01)     **A61B 6/03** (2006.01)

(52) Cooperative Patent Classification (CPC):
**G16H 40/63; A61B 6/46**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **WEISS, Steffen**
**5656 AE Eindhoven (NL)**

• **LEUSSLER, Christoph Günther**
**5656 AE Eindhoven (NL)**
• **VOGTMEIER, Gereon**
**5656 AE Eindhoven (NL)**
• **JOHNSON, Mark Thomas**
**5656 AE Eindhoven (NL)**

(74) Representative: **Philips Intellectual Property & Standards**
**High Tech Campus 52**
**5656 AG Eindhoven (NL)**

(54) **AN ENTERTAINMENT CONTENT PROVIDER FOR USE DURING A MEDICAL PROCEDURE**

(57)     This invention provides a system for scheduling of audio tracks, wherein the audio tracks are stored with data relating to the duration but also the loudness level of the audio track. A sequence of audio tracks is generated which aims to match the audio track loudness levels to the noise levels of a scanning step to be performed at the same time, as well as matching the audio track durations to the scanning step durations.

FIG. 2

**Description**

FIELD OF THE INVENTION

The present invention generally relates to an entertainment content provider and corresponding method and computer program product for providing an entertainment sequence to a patient during a medical procedure.

BACKGROUND OF THE INVENTION

To reduce a patient's anxiety and improve their experience during a medical procedure, entertainment may be provided during the medical procedure, such as audio (music, audiobooks), calming images or a movie, for example, on an entertainment screen or through goggles.

The medical procedure is for example Magnetic Resonance Imaging (MRI) or other (tomographic and non-tomographic) diagnostic imaging scan (e.g. X-ray imaging, computed tomography, positron emission tomography, single-photon emission computed tomography, ultrasound imaging, etc.), image-guided treatment or therapies, radiotherapy procedures, proton therapy or some interventional procedures.

The patient entertainment typically consists of audio playback of play lists chosen by staff or the patient. Sometimes, video content is displayed on a screen at the rear end of the scanner and observed by the patient via a mirror.

By way of example, a magnetic resonance (MR) examination consists of a schedule of several scanning steps (which may be complete scans or portions of a scan) with different durations and levels of MR noise. Often, some form of communication is performed between these scanning steps, e.g. to announce the next scanning steps, to state its duration, to remind the patient to be patient or lie still, or to ask for preparation of a breath-hold. Some or all of these commands are automatized.

Audio songs generally do not fit into the schedule defined by the sequence of scanning steps with communication in-between. Similarly, movies generally do not match the duration of an entire MR examination. This interference with the MR examination lowers the quality of patient entertainment. For example, songs are interrupted by breaks between scanning steps and the patient may be disturbed by particularly loud scanning steps when listening to quiet songs. Movies are often interrupted at unfavorable points in time or patients frequently miss the end of their movie.

SUMMARY OF THE INVENTION

The invention is defined by the claims.

According to examples in accordance with an aspect of the invention, there is provided an entertainment content provider for providing an entertainment sequence to a patient during a medical scan, wherein the medical scan comprises a succession of scanning steps each with a respective duration and noise level, comprising:

a database containing a plurality of audio tracks, each audio track being stored with data relating to the duration and loudness level of the audio track;
a scheduling system for creating a schedule of audio tracks to be played to a patient during a medical scan, wherein the scheduling system is adapted to:

receive a planned sequence of the scanning steps including their durations and noise levels; and
determine a sequence of audio tracks which aims to match the audio track loudness levels to the scanning step noise levels and to match the audio track durations to the scanning step durations.

This content provider selects (or adapts) audio tracks such that their duration and loudness are matched to scanning step durations and noise levels. A medical scan, such as an MR examination, consists of several scanning steps with different durations and levels of MR noise, for example interrupted by communication with the patient, e.g. to remind to lie still, or to ask for a breath-hold. The invention avoids interruption to audio tracks as well as matching the loudness levels, by reordering audio play lists so that louder and longer songs are played back during louder and longer scanning steps.

The audio content may be adapted for all parts of a scan, including phases before and after entering the scanning bore.

The audio tracks may be selected by a user, or only a genre of audio tracks may be selected by the user (and the system then selects from a list of tracks of that genre), or the user may select a predefined playlist of audio tracks. The system determines the order of the tracks as well as any adaptation of the tracks themselves, if needed to provide better matching to the scan procedure.

The scheduling system may be adapted to adapt the length of an audio track during the course of a medical scan, thereby to adapt in real time to changes in the planned sequence of scanning steps or changes in the durations of the scanning steps.

The audio tracks may for example be adapted in duration to match the durations of scan steps if interaction with the patient is required in-between.

Some scans often are performed with respiratory gating and/or cardiac gating. These scans do not have a fixed duration but vary in length depending on the respiratory or cardiac cycle length, number of missed gating signals, gating efficiency and further unforeseeable parameters. The real time adaptation enables the scheduling to adapt in response to these scan characteristics.

The scheduling system may be adapted to receive from a medical scanning system a real-time forecast of the remaining duration of the scanning steps. This enables the real-time adaptation to follow up-to-date forecast information.

The scheduling system may be adapted to perform a remix function to cut or add audio parts and stitch audio parts together to adapt the audio track length.

State-of-the-art software (such as Adobe Premiere Pro and Adobe Audition) are known with this remix functionality. The remix functionality may even create new audio tracks as a mixture of known elements from different audio tracks for better matching with a scan sequence. The remix function may also adapt a beat per minute tempo without changing tone height.

The scheduling system may be adapted to:

derive a dissimilarity function which is based on:

differences between the durations of the audio tracks and scanning steps; and
differences between the loudness levels of the audio tracks and noise levels of the scanning steps; and

determine a sequence of audio tracks which minimizes an overall dissimilarity value.

Thus, an algorithm is used to determine a sequence which best matches the scanning steps.

The scheduling system may be further adapted to modify the audio tracks to provide breathing guidance. The audio tracks may thus additionally be modified, to encode breathing guidance instructions onto the audio track. This may involve providing a periodic modulation of an audio parameter.

The audio parameter may be an intensity, pitch, timbre, or dynamic range for example, and the patient is encouraged to follow the rhythm. Encouragement can be provided by prior instruction or training, or communicated at those points of the scan where breathing gating is carried out. The periodic modulation may be chosen to fit naturally to the characteristics of the track (breathe in for one phrase of music, breathe out for the next phrase).

By controlling the breathing of the patient, the duration of a breathing-gated scan becomes more predictable and hence the adaptation of the audio tracks becomes easier.

The scheduling system may be adapted to complete the modulation of an audio parameter before the end of the track, based on the known desired duration of the breathing guidance and the length of the track.

Preferably, the guidance should be timed such to provide some additional time at the end of the track to accommodate any non-adherence to the breathing guidance. The scheduling system for example comprises a machine learning neural network.

The database may further contain a plurality of videos, each video being stored with data relating at least to the duration of the video, wherein the scheduling system is further for adapting a chosen video to match an overall scan duration. Thus, in addition to audio track scheduling, the system can also provide adaptation of video content to match a scan duration.

The scheduling system is for example adapted to make a selection of sections between cuts of the chosen video thereby to adapt the chosen video to match the overall scan duration.

Each video may include an audio track, and the scheduling system is further adapted to reorder the video thereby to match the audio track loudness levels to the scanning step noise levels. Thus, the loudness matching function of the invention may also be applied to video content.

The scheduling system may also be adapted to modify the video to provide breathing guidance by providing a periodic modulation of a video parameter.

The medical scan for example comprises a magnetic resonance scan, a computed tomography scan, a positron emission tomography scan, a single-photon emission computed tomography scan, an ultrasound scan, or fluoroscopy.

The invention also provides a method for adaptively providing an entertainment sequence to a patient during a medical scan, wherein the medical scan comprises a succession of scanning steps each with a respective duration and noise level, the method comprising:

selecting an entertainment option from an entertainment content database comprising a plurality of audio tracks, each audio track being stored with data relating to the duration and loudness level of the audio track; and creating a schedule of audio tracks to be played to a patient during a medical scan by:

receiving a planned sequence of the scanning steps including their durations and noise levels; and determining a sequence of audio tracks which aims to match the audio track loudness levels to the scanning step noise levels and to match the audio track durations to the scanning step durations.

The length of an audio track may be adapted during the course of the medical scan, thereby to adapt in real time to changes in the planned sequence of scanning steps or changes in the durations of the scanning steps.

The invention also provides a computer program comprising computer program code which is adapted, when said program is run on a processor, to perform the steps of the method define above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig.1 shows an MR scanner with an entertainment system, in which figure la shows a schematic set-up and figure 1b shows a photograph of a patient inside a bore of the MR scanner.
Fig. 2 shows an entertainment content provider for providing an entertainment sequence.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for scheduling of audio tracks, wherein the audio tracks are stored with data relating to the duration but also the loudness level of the audio track. A sequence of audio tracks is generated which aims to match the audio track loudness levels to the noise levels of a scanning step to be performed at the same time, as well as matching the audio track durations to the scanning step durations.

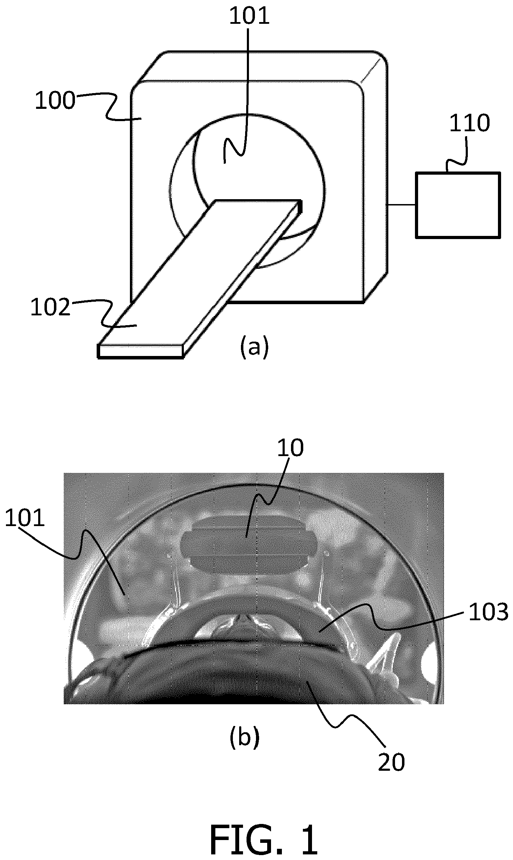

Fig. 1 shows an MRI device 100 for receiving a patient 20 on a patient support 102 in the bore 101 of the MRI device 100. The MRI device uses strong magnetic fields, magnetic field gradients and radio waves (picked up by receiving coils 103) and a processing unit 110 to generate images of the organs in the patient's body. A schematic set-up is shown in Fig. 1a and photograph of a patient 20 inside a bore 101 is shown in Fig. 1b.

As is immediately clear from Fig. 1b, the bore 101 is relatively small and, in the example shown, a head coil 103 is placed closely over the patient's head, creating a very narrow space that may induce claustrophobic feelings in the patient. Furthermore, the MRI scan may take between a few minutes to even an hour an half or more in which the patient has to lie still, while the procedure involves regular and irregular loud noises close to the patient's ears. All these factors contribute to the patient feeling uncomfortable, claustrophobic, impatient or even scared. This is particularly relevant for pediatric or psychiatric patients.

To provide distraction and relief an in-bore entertainment system 10 that provides entertainment to the patient during the scan may be used. The entertainment system may for instance be an in-bore display or a mirror or screen onto which the entertainment content is projected. The entertainment system 10 usually includes headphones (not shown), which also reduce ambient noises and may be used to communicate with the patient 20. The entertainment system 10 may also include means for providing visual information and entertainment, such as an (MRI-compatible) in-bore screen, a screen placed outside of the bore that is seen through a mirror, a screen on the ceiling of the imaging room, 2D or 3D (MRI-compatible) goggles or a projected screen, The display function may also be used to provide the patient with instructions and/or information relating to the scan (e.g. time left, a scan progress bar) as well as entertainment.

As mentioned above, it may be problematic when the length of the entertainment is not immersive and/or has is not of the same length as the length of the procedure, which may actually contribute to a poor patient experience and the risk of sub-optimal or even non-usable scan results causing a rescan or even a misdiagnosis. While many procedures may be planned and performed according to plan (which may be followed using an Exam Card that includes all the relevant and necessary scan data), it may occur regularly that during the scan the plan is adapted and the Exam Card is updated. For instance, a running procedure may be lengthened because a (partial) rescan may need to be done in case of patient movement or another irregularity or an additional scan may be planned if during the procedure additional information is obtained from the ongoing scan(s). Alternatively, an ongoing procedure may be shortened, for instance when a patient needs additional instructions during the scan that may pause the entertainment (thereby shortening the remaining time to provide the entertainment) or a planned follow-up scan may be cancelled due to additional information obtained from the ongoing scan(s).

The applicant has proposed, but not yet published, an adaptive entertainment content provider that ensures that the length of the provided entertainment matches the procedure length, even if the procedure length is either lengthened or shortened during the medical procedure. This makes the patient's experience more satisfying, calming and/or bearable. This directly results in increased image quality, as movement is likely to be much less. This results in lower change of misdiagnoses and/or fewer procedures that need to be aborted and restarted due to disruptions caused by a patient's adverse mental state (e.g. anxiety, boredom, annoyance or any other negative mental state that may cause movement).

The previous proposal is based on an insight that most video entertainment has a sequence of entertainment sections with some sections that are essential to tell a story or provide a full experience and other sections that are not essential to the central plot, but do enrich the story by providing more context, side-stories and/or deepen character characteristics. As such, a full entertainment sequence may be sub-divided into several blocks and each block may be flagged as being essential or as being non-essential. Entertainment content of various lengths may be built up using different combinations of the blocks.

This approach ensures that entertainment content has a suitable duration relative to the scan.

This invention relates in particular to the scheduling of audio tracks, wherein the audio tracks are stored with data relating to the duration but also the loudness level of the audio track. As mentioned above, a sequence of audio tracks is generated which takes account of the audio track loudness levels and the noise levels of the scanning steps.

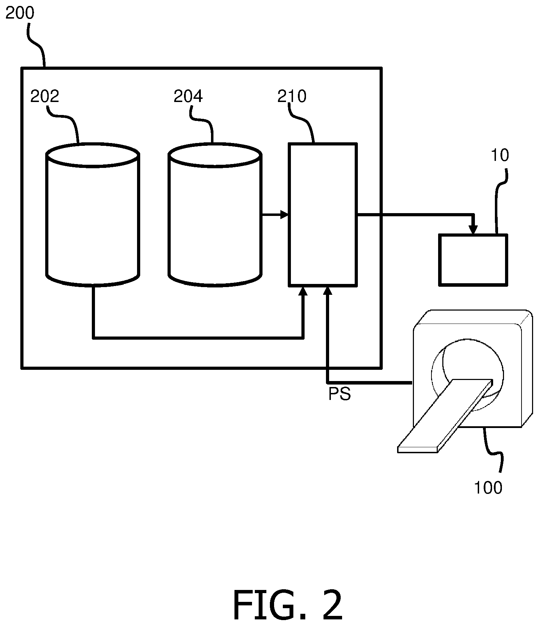

Fig. 2 shows an entertainment content provider 200 for providing an entertainment sequence to a patient during a medical scan. The content provider comprises a database 202 containing a plurality of audio tracks, each audio track being stored with data relating to the duration and loudness level of the audio track. The loudness may be a minimum loudness level of the audio track (which then determines how little noise is needed to be able to hear all of the track), or a general loudness level of the track (which determines how little noise is needed to generally hear the track overall), or it may be a loudness function over time for the audio track.

There is also an optional database 204 of video content, comprising paired audio tracks and video tracks, again with loudness data in respect of the audio tracks as well as duration information.

A scheduling system 210 is for creating a schedule of audio tracks (which may have associated video tracks as well) to be played to a patient during a medical scan. The scheduling system is basically a processor which receives a planned sequence PS of the scanning steps including their durations and noise levels. Using this planned sequence, and duration and loudness data of the audio tracks, it determines a sequence of audio tracks which aims to match the audio track loudness levels to the scanning step noise levels and to match the audio track durations to the scanning step durations.

This "matching" involves finding a most suitable timing for different audio tracks relative to the scanning steps of the medical scan. It does not mean noise levels and track loudness are chosen to be the same, rather it means a most suitable pairing (subject to various constraints such as the actual audio tracks themselves) is created between the scanning steps and the audio tracks.

The matching for example involves reordering of audio play lists (and optionally also video tracks) based on loudness and duration. The noise level of each scanning step in an MR examination is already determined by state-of-the-art MR systems and normalized to the loudest scan to determine a normalized loudness. This is already available data from an MR system. Similarly, the sound level of all songs in a play list (or video parts in a video) can be analyzed using known methods in audio processing and again can be normalized to a loudest song.

The scheduling system may receive a set of selected audio tracks, either chosen by the patient, or forming a predefined playlist chosen by the patient, or the patient may simply select a genre and the system is then able to create a sequence of audio tracks from a much larger set of tracks of that genre. A playlist preferably includes more tracks than are needed to fill the scan time, so that the system has more flexibility in choosing a combination of audio tracks that best matches the scanning steps.

One way to perform the matching is to define a dissimilarity function D with normalized loudness and duration of songs and scanning steps as input, and a dissimilarity measure as output. For each scanning step, the audio track is chosen (or adapted) that minimizes this function D. The function may be defined as:

$$D(v_M, d_M, v_S, d_S) = a * 100 * abs(d_M - d_S)^n / min(d_M, d_S) + (1-a) * abs(v_M - v_S)$$

with normalized volume of song $v_M$ (assuming a single loudness level for an entire audio track), duration of song $d_M$, normalized volume of scan vs, duration of scan ds, a is a weighting factor in the order of 0.5 (range [0..1]), and an exponent n is in the range [1..3].

The function D is thus based on differences between the loudness levels of the audio tracks and noise levels of the scanning steps.

With n=2 a mismatch in duration is quadratically "punished", which limits the problem of extraordinate shortening or extension of a song. The first term (without factor a) is about 1, 4, 9 for a relative difference of 10%, 20%, 30% of the durations respetively.

Effectively, this function ranges between 0 and the order of 10 for reasonable input values. D may be defined differently to put more or less emphasis on matching duration or volume.

Audio tracks may be shortened or lengthened automatically and on the fly during an examination to adapt to the durations of scanning steps if interaction with the patient is required in-between. State-of-the-art software as Adobe Premiere Pro and Adobe Audition can be used for this purpose. These provide remix functions that can cut or add audio parts automatically and stitch parts together seamlessly to match the duration of a song to a desired duration, in this case the duration of a scanning step.

Many scans, such as of the thorax and abdominal regions, are often performed with respiratory gating and/or cardiac gating. These scans do not have a fixed duration but vary in real time in length depending on the respiratory and cardiac cycle length, the number of missed triggers, the gating efficiency and further unforeseeable parameters. A real-time forecast of the remaining duration of these scans is however provided by known MR scanners. In the system of the invention, this information is used to adapt media on the fly to end with the scan.

The acoustic noise level of each scan in an MR examination is determined by state-of-the-art MR systems and normalized to the loudest scan. The audio to be provided may be processed in combination with this noise level data of the scan-protocol for adapting the audio level.

If an anti-noise system is integrated into the system, a level of noise reduction can depend on the scan parameters. A noise reduction system may thus be used to reduce the dynamic range for the adaptation of the audio level. The noise reduction can for example be switched on when the MR sequence has a high noise level and switched off for a low noise level.

Thus, during the scan, a noise reduction function can be switched on and off or adapted. This also allows acoustic relatively quiet periods to be provided between the audio content, for example at times when the patient has to concentrate or needs to follow guidance as is for example needed for functional imaging. In this way, the system allows acoustic blanking to be integrated with the audio content, which can be beneficial in some clinical protocols.

Another option is to provide breathing guidance by adapting audio content (and optionally also video content), for example by providing a periodic modulation in an audio parameter (intensity, pitch, timbre, dynamic range etc.) which the patient is encouraged to follow. Encouragement can be provided by prior instruction or training, or communicated at those points of the scan where breathing gating is carried out.

The advantage of such an approach is that a compliant patient will breathe in a guided manner and as such the duration of the scan becomes more predictable. As a consequence, the periodic modulation of the track can be chosen to fit more naturally to the details of the track (e.g. breathe in for one phrase of music, breathe out for the next phrase). It can also be ensured that the modulation is completed before the end of the track by knowing the duration of the breathing guidance and the length of the track (which should exceed the duration of guidance). Preferably, the guidance should be timed such as to provide some additional time at the end of the track to accommodate any non-adherence to the breathing guidance.

Video content stored in the database 204 may also be adapted to assist the patient in breathing, for example by providing a periodic modulation in the video track of a video parameter (intensity, color rendition, ambient lighting intensity or color palate etc.). The audio track of video content may be adapted in the same way as an audio track alone, by adapting an audio parameter of the corresponding audio soundtrack (intensity, pitch, timbre, dynamic range etc.) which the patient is encouraged to follow.

As for the audio adaptation, adaptation of video content can be selected so that guidance fits naturally to the details of the video (breathe in for one sequence, breathe out for the next sequence, breathe out with one character in screen, breathe out when another character is on screen). The modulation can be completed before the end of the video by knowing the duration of the breathing guidance and the length of the video (which should exceed the duration of guidance).

The processing of audio tracks may further involve adapting or even removing certain audio features of the audio tracks, such as changing the melody, rhythm or base. This function can be implemented using existing audio processing software. By way of example, some songs, such as HIP Pop songs, have a clear rhythm but it may be beneficial to remove the vocal tracks. The song is then more compatible with the clinical situation, but still gives a positive ambiance in the scanner.

Different instruments like a cow bell or a drum beat (such as a hi hat, cymbal drum) can be used to trigger and control the patient. Certain song texts and vocals can be used for triggering and control of breathing or other actions. A cow bell sound may be directly linked to a breathing or motion rhythm.

As another option, audio tracks may be composed specifically for this particular use. For example, time stamps may be added to tracks which can be activated. For example, a song may have several pause signs which can be stretched or shorted. Individual bars may be periodically removed and replaced by a rhythm beat for triggering purposes. Instruments or sounds may be removed and replaced to better match the scan.

The scan sound itself has a melody, and music can be composed to adapt the instruments to the scan melody. An AI based system may for example automatically compose or modify the melody or sound of a song such that a more patient friendly result is achieved having regard to the sounds or melody or rhythm of the scan sounds.

An audio remixing function may also be used to create new songs or audio tracks as a mixture of known elements from different audio tracks into a new scan sequence. An audio track consists of several themes that are combined in a certain sequence and the theme sometimes comes back in a modified version along the audio track. If the scan sequences are considered themselves as audio themes, then also the composition of the complete imaging sequence can be considered to correspond to a composition of several audio tracks. To have enough variation and also to consider personal preferences, there may be a set of base audio tracks that match a scan sequence and could be randomly selected. Repeated scan sequences may then lead to repeated audio tracks but with the option of slightly modified themes but still recognizable to the listening patient.

Individual scan parameters leading to louder scan sequences can be translated to more dynamic and louder audio sub-tracks within the matching audio track, using crescendo and decrescendo passages. Beat synchronization with MRI sequence coil noise can also be employed. Tools to transform an audio sequence to adapt to a desired beat per minute (bmp) are also known which avoid changing the tone height. Even a patient-specific remix may be created for a follow up imaging procedure so that comparable results are achieved with the same scanner settings and close to identical environmental conditions. This could improve repeatability of a diagnostic scan.

The adaptation of an audio sequence may also be made to achieve a certain purpose (e.g. to gain attention for the start of a new sequence). This may be based on specific audio tracks that are repeated whenever the patient has to react in the same way. Artificial audio composing tools can be used to produce the audio track.

A library may be formed of audio tracks with different beat patterns, suitable for use with particular scan sequences. The beat may be matched to a beat of the scan sequence. The library may be tailored to the preferences of the audio preference of individuals for example based on their playlists. Patient musical preferences may be used to influence the selection of the matching audio tracks - for example selecting between classical music, jazz music, synthesizer sounds or other styles. A patient's preferences may be checked from a user profile or from a patient specific scan preparation.

The audio content may be adapted to all parts of the examination, including phases before and after entering the bore, and thus not limited to the diagnostic scan itself. The patient for example spends time undressing, redressing, being instructed, being prepared with sensors, having sensors removed, awaiting images etc.

A machine learning neural network may be used for processing the audio to be provided, for example based on the audio signal and the noise data of the scan protocol.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. An entertainment content provider for providing an entertainment sequence to a patient during a medical scan, wherein the medical scan comprises a succession of scanning steps each with a respective duration and noise level, comprising:

a database (202,204) containing a plurality of audio tracks, each audio track being stored with data relating to the duration and loudness level of the audio track;
a scheduling system (210) for creating a schedule of audio tracks to be played to a patient during a medical scan, wherein the scheduling system is adapted to:

receive a planned sequence of the scanning steps including their durations and noise levels; and
determine a sequence of audio tracks which aims to match the audio track loudness levels to the scanning step noise levels and to match the audio track durations to the scanning step durations.

2. The content provider of claim 1, wherein the scheduling system (210) is adapted to adapt the length of an audio track during the course of a medical scan, thereby to adapt in real time to changes in the planned sequence of scanning steps or changes in the durations of the scanning steps.

3. The content provider of claim 2, wherein the scheduling system (210) is adapted to receive from a medical scanning system a real-time forecast of the remaining duration of the scanning steps.

4. The content provider of claim 2 or 3, wherein the scheduling system (210) is adapted to perform a remix function to cut or add audio parts and stitch audio parts together to adapt the audio track length.

5. The content provider of any one of claims 1 to 4, wherein the scheduling system (210) is adapted to:

derive a dissimilarity function which is based on:

differences between the durations of the audio tracks and scanning steps; and
differences between the loudness levels of the audio tracks and noise levels of the scanning steps; and

determine a sequence of audio tracks which minimizes an overall dissimilarity value.

6. The content provider of any one of claims 1 to 5, wherein the scheduling system (210) is further adapted to modify the audio tracks to provide breathing guidance.

7. The content provider of claim 6, wherein the scheduling system (210) is adapted to modify the audio tracks to provide breathing guidance by providing a periodic modulation of an audio parameter.

8. The content provider of claim 7, wherein the scheduling system (210) is adapted to complete the modulation of an audio parameter before the end of the track, based on the known desired duration of the breathing guidance and the length of the track.

9. The content provider of any one of claims 1 to 8, wherein the database (202,204) further contains a plurality of videos, each video being stored with data relating at least to the duration of the video, wherein the scheduling system (210) is further for adapting a chosen video to match an overall scan duration.

10. The content provider of claim 9, wherein each video includes an audio track, wherein the scheduling system is further adapted to reorder the video thereby to match the audio track loudness levels to the scanning step noise levels.

11. The content provider of claim 9 or 10, wherein the scheduling system (210) is adapted to modify the video to provide breathing guidance by providing a periodic modulation of a video parameter.

12. The content provider of any one of claims 1 to 11, wherein the medical scan comprises a magnetic resonance scan, a computed tomography scan, a positron emission tomography scan, a single-photon emission computed tomography scan, an ultrasound scan, or fluoroscopy.

13. A method for adaptively providing an entertainment sequence to a patient during a medical scan, wherein the medical scan comprises a succession of scanning steps each with a respective duration and noise level, the method comprising:

selecting an entertainment option from an entertainment content database comprising a plurality of audio tracks, each audio track being stored with data relating to the duration and loudness level of the audio track; and creating a schedule of audio tracks to be played to a patient during a medical scan by:

receiving a planned sequence of the scanning steps including their durations and noise levels; and determining a sequence of audio tracks which aims to match the audio track loudness levels to the scanning step noise levels and to match the audio track durations to the scanning step durations.

14. The method of claim 13, comprising adapting the length of an audio track during the course of the medical scan, thereby to adapt in real time to changes in the planned sequence of scanning steps or changes in the durations of the scanning steps.

15. A computer program comprising computer program code which is adapted, when said program is run on a processor, to perform the steps of the method of claim 13 or 14.

101

100

110

102

(a)

10

101

103

20

(b)

# FIG. 1

# FIG. 2

Europäisches
Patentamt

European
Patent Office

Office européen
des brevets

# EUROPEAN SEARCH REPORT

Application Number

EP 21 18 0215

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2011/142250 A1 (SCHMALE INGO [DE] ET AL) 16 June 2011 (2011-06-16) * [0005], [0016], [0023]-[0026], [0038] * | 1-15 | INV. G16H40/63 A61B6/03 |
| Y | US 9 131 205 B2 (GILLIES MURRAY FULTON [NL]; VAN EE RAYMOND [NL] ET AL.) 8 September 2015 (2015-09-08) * col 2 lines 9-49, col 3 line 46 - col 4 line 5, col 7 last par * | 1-15 | |
| Y | DAN MA ET AL: "Music-based magnetic resonance fingerprinting to improve patient comfort during MRI examinations", MAGNETIC RESONANCE IN MEDICINE, vol. 75, no. 6, 16 July 2015 (2015-07-16), pages 2303-2314, XP055314061, ISSN: 0740-3194, DOI: 10.1002/mrm.25818 | 4 | |
| A | * page 2308 right col par 3 * | 1-3,5-15 | |
| A | US 6 463 316 B1 (BRUNGART DOUGLAS S [US]) 8 October 2002 (2002-10-08) * col 4 lines 11-49 * | 1-15 | TECHNICAL FIELDS SEARCHED (IPC) G16H A61B |

The present search report has been drawn up for all claims

| Place of search | Date of completion of the search | Examiner |
|---|---|---|
| Munich | 2 December 2021 | Bankwitz, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 21 18 0215

This annex lists the patent family members relating to the patent documents cited in the above-mentioned European search report.
The members are as contained in the European Patent Office EDP file on
The European Patent Office is in no way liable for these particulars which are merely given for the purpose of information.

02-12-2021

| Patent document cited in search report | | Publication date | Patent family member(s) | | Publication date |
|---|---|---|---|---|---|
| US 2011142250 | A1 | 16-06-2011 | CN | 102119339 A | 06-07-2011 |
| | | | EP | 2313794 A1 | 27-04-2011 |
| | | | JP | 2011530371 A | 22-12-2011 |
| | | | US | 2011142250 A1 | 16-06-2011 |
| | | | WO | 2010018534 A1 | 18-02-2010 |
| US 9131205 | B2 | 08-09-2015 | CN | 103228316 A | 31-07-2013 |
| | | | EP | 2643043 A1 | 02-10-2013 |
| | | | US | 2013245364 A1 | 19-09-2013 |
| | | | WO | 2012069979 A1 | 31-05-2012 |
| US 6463316 | B1 | 08-10-2002 | NONE | | |

EPO FORM P0459

For more details about this annex : see Official Journal of the European Patent Office, No. 12/82